# EUROPÄISCHE PATENTSCHRIFT

(11) **EP 3 257 530 B1**
(45) Veröffentlichungstag und Bekanntmachung des Hinweises auf die Patenterteilung: **09.01.2019**
(21) Anmeldenummer: 17173023.7
(22) Anmeldetag: 26.05.2017
(51) Int. Cl.: A61L 29/08, A61L 29/12, A61L 29/16, C08L 29/04, C08L 71/02

(54) **BESCHICHTETER BALLONKATHETER UND ZUSAMMENSETZUNG ZUR BESCHICHTUNG DES BALLONKATHETERS**
COATED BALLOON CATHETER AND COMPOSITION FOR COATING THE BALLOON CATHETER
CATHÉTER À BALLONNET REVÊTU ET COMPOSITION DE REVÊTEMENT DU CATHÉTER À BALLONNET

(30) Priorität: 13.06.2016 DE 102016110815
(43) Veröffentlichungstag der Anmeldung: 20.12.2017
(73) Patentinhaber: DOT GmbH, 18059 Rostock (DE)
(72) Erfinder: NEUMANN, Hans-Georg, 18146 Rostock (DE); BUHRMEISTER, Mischa, 18059 Papendorf (DE)
(74) Vertreter: Prinz & Partner mbB

(56) Entgegenhaltungen:
- EP-A1- 2 643 030

## Beschreibung

### GEBIET DER ERFINDUNG

Die Erfindung betrifft einen beschichteten Ballonkatheter und eine Zusammensetzung zur Beschichtung eines Ballonkatheters.

### HINTERGRUND DER ERFINDUNG

Ballonkatheter dienen zur Aufweitung krankhaft verengter Blutgefäße (Stenosen). Der an einem Gefäßkatheter angebrachte Ballon wird über eine Arterie, beispielsweise die Beinschlagader, eingeführt und unter Röntgenkontrolle zur verengten Gefäßstelle vorgeschoben. Dort wird der Ballon langsam unter Druck entfaltet. Die Verengung wird dadurch erweitert und ein ungestörter Blutfluss ermöglicht.

Um eine erneute Verengung zu verhindern, kann zusätzlich ein Stent implantiert werden. Abhängig vom Stenoseort, der Gefäßgröße und Vorerkrankungen können mit Medikamenten beschichtete Stents verwendet werden.

Bei einigen Patienten kommt es nach der Aufweitung der Stenosen schon nach wenigen Monaten zu einer erneuten Verengung (Restenose). Die Restenose kann auf eine übermäßige Proliferation insbesondere der glatten Muskelzellen zurückgeführt werden, die durch Verletzungen aufgrund der gewaltsamen Aufweitung der Gefäßwände ausgelöst wird. Nach Abheilung der Verletzungen kommt die Proliferation nicht sofort zum Stillstand und führt so häufig zu einer Restenose. Zur Verhinderung einer Restenose wurden mit Medikamenten beschichtete Ballonkatheter, sogenannte "Drug Eluting Ballons", entwickelt. Ein häufig verwendeter Wirkstoff mit anti-proliferativer Wirkung ist Paclitaxel.

Mit Medikamenten beschichtete Ballonkatheter können außerdem dazu genutzt werden, eine lokale Arzneimittelgabe an die Gefäßwand zu bewirken, ohne dass eine Weitung der Gefäße erfolgt, beispielsweise im Falle einer Behandlung von Veränderungen der Gefäßwand, die nicht mit einer Stenose verbunden sind (z. B. vulnerable Plaques, aufgelagerte Thromben), oder bei der Behandlung von Gefäßen mit mechanischen Mitteln oder mit thermischen Verfahren. In diesen Fällen liegt der Ballon nicht vollständig an den unregelmäßigen Gefäßwänden an und der Wirkstoff kann nur an der Kontaktstelle übertragen werden. Dazu ist eine möglichst gleichmäßige Beschichtung der Ballonoberfläche erwünscht.

Beschichtete Ballonkatheter sind aus der WO 2005/089855 A1 bekannt. Die WO 2004/028582 A1 offenbart mehrfach gefaltete Ballons, die mit einer Zusammensetzung eines pharmakologischen Wirkstoffs und einem Kontrastmittel beschichtet sind, vorzugsweise innerhalb der Falten. Eine Methode zum Sprühbeschichten von Ballonkathetern ist in der WO 2004/006976 A1 beschrieben.

Aus der DE 20 2010 017 248 U1 ist ein Ballonkatheter bekannt, der eine Beschichtung aus Paclitaxel und Schellack aufweist. Die Verwendung von Schellack als Bindemittel soll während der kurzen Kontaktzeit des Ballonkatheters mit der Gefäßwand eine schnelle Freisetzung des Wirkstoffs Paclitaxel begünstigen.

Während der Entfaltung des Ballonkatheters beträgt die Kontaktzeit des Ballons mit der Gefäßwand am Stenoseort nur einige Sekunden bis wenige Minuten. Daher ist eine gleichmäßige Beschichtung der Ballonoberfläche vorteilhaft, damit die Kontaktfläche des Ballons mit der Gefäßwand möglichst vollständig zur Wirkstoffabgabe genutzt werden kann. Gleichzeitig muss die Beschichtung hinreichend stabil sein, damit sie während des Transports durch die Blutgefäße bestehen bleibt.

Die derzeit zugelassenen, mit einer Beschichtung versehenen Ballonkatheter haben das Problem, dass ein beträchtlicher Teil der Beschichtung sich auf dem Weg zum Zielort ablöst, so dass eine zu geringe Menge des Wirkstoffs seinen Wirkungsort erreicht. Diesem Problem wird häufig dadurch entgegengewirkt, dass ein Mehrschichtsystem auf den Ballon aufgetragen wird, das eine oder mehrere Adhäsionsschichten zur Verbesserung der Haftung auf der Ballonoberfläche, die eigentliche Wirkstoffschicht sowie eine oder mehrere Deckschichten zur Verminderung der Ablösegefahr der Wirkstoffschicht beinhaltet.

Die WO 2014/029442 beschreibt einen solchen Ballonkatheter mit einer Beschichtung, die wenigstens eine Schicht mit einem antiproliferativen, immunsuppressiven, anti-angiogenen, anti-inflammatorischen, anti-restenotischen und/oder anti-thrombotischen Wirkstoff und eine wirkstofffreie Deckschicht aus einem Polyvinylalkohol-Polyethylenglycol-Propfcopolymer umfasst.

Das Vorhandensein einer Deckschicht über der wirkstoffhaltigen Lage auf der Ballonoberfläche kann jedoch dazu führen, dass eine unzureichende Wirkstoffmenge an die Gefäßwand abgegeben wird, insbesondere wenn die Ballonoberfläche nur teilweise in Kontakt mit der zu behandelnden Gefäßwand steht. Ohne die Deckschicht treten aber schon beim Einführen des Ballonkatheters in die Gefäße bis zum Stenoseort erhebliche Wirkstoffverluste auf.

### ZUSAMMENFASSUNG DER ERFINDUNG

Der Erfindung liegt daher die Aufgabe zugrunde, einen Ballonkatheter mit stabiler Beschichtung anzugeben, der eine möglichst homogene Wirkstoffverteilung auf der Ballonoberfläche des Katheters aufweist und eine gesteuerte Wirkstoffabgabe an die Gefäßwand am Stenoseort ermöglicht.

Erfindungsgemäß wird diese Aufgabe durch einen Ballonkatheter nach Anspruch 1 gelöst.

Vorteilhafte Ausführungsformen der Erfindung sind in den Unteransprüchen angegeben, die wahlweise miteinander kombiniert werden können.

Der beschichtete Ballonkatheter umfasst ein Kathetersubstrat und einer Beschichtung auf dem Kathetersubstrat. Die Beschichtung umfasst einen in einer Bindermatrix eingebetteten pharmazeutisch aktiven Wirkstoff. Erfindungsgemäß besteht die Bindermatrix aus einem Polyethylenglycol-Polyvinylalkohol-Copolymer (PEG-PVA-Copolymer) und wahlweise Schellack. Ferner kann die Beschichtung weitere pharmazeutisch verträgliche Hilfsstoffe umfassen.

Die Verwendung einer Bindermatrix mit einem Anteil an PEG-PVA-Copolymer führt zu mechanisch ausreichend stabilen Beschichtungen, aus denen während des Einführens des Katheters in die Gefäße nur wenig Wirkstoff abgelöst wird. Dennoch sind die Beschichtungen so beschaffen, dass auch bei den kurzen Kontaktzeiten der Ballonbeschichtung mit den Gefäßwänden am Stenoseort eine ausreichend hohe Menge des pharmazeutisch aktiven Wirkstoffs abgegeben wird.

Die meisten pharmazeutischen Wirkstoffe mit proliferativer Wirkung sind jedoch nicht oder nur schwer löslich in Wasser, während das im Stand der Technik bereits als schützende Deckschicht eingesetzte PEG-PVA-Copolymer nur als wasserlöslich beschrieben ist. Die Erfinder haben jetzt erkannt, dass eine stabile Beschichtung mit PEG-PVA-Copolymer als Bindemittel und den in Wasser nicht löslichen antiproliferativen Wirkstoffen wie Paclitaxel durch eine selektive Auswahl der für die Beschichtungslösung verwendeten Lösungsmittel und deren Abfolge beim Auflösen des Bindemittels und des Wirkstoffs hergestellt werden kann. Überraschenderweise kann durch die Wahl der Lösungsmittel auch die Strukturierung des Wirkstoffs in der Beschichtung und damit die Verfügbarkeit des Wirkstoffs am Stenoseort eingestellt und gesteuert werden.

Gegenstand der Erfindung ist somit auch eine Zusammensetzung zur Beschichtung eines Ballonkatheters, wobei die Zusammensetzung einen pharmazeutisch aktiven Wirkstoff und ein Bindemittel umfasst, das aus einem PEG-PVA-Copolymer und wahlweise Schellack besteht, wobei der pharmazeutisch aktive Wirkstoff und das Bindemittel in einem Gemisch von Wasser und wenigstens einem weiteren mit Wasser unbegrenzt mischbaren organischen Lösungsmittel gelöst sind.

Ferner betrifft die Erfindung ein Verfahren zur Herstellung eines beschichteten Ballonkatheters, welches die folgenden Schritte umfasst:
a) Bereitstellen einer wässrigen Lösung eines PEG-PVA-Copolymers;
b) schrittweise Versetzen der wässrigen Lösung von Schritt a) mit wenigstens einem weiteren, mit Wasser unbegrenzt mischbaren organischen Lösungsmittel unter Bildung einer wässrig-organischen Lösung;
c) Mischen der wässrig-organischen Lösung mit einem pharmazeutisch aktiven Wirkstoff und homogenisieren des Gemischs unter Bildung einer wirkstoffhaltigen Beschichtungslösung;
d) wahlweise Zugeben von DMSO in einem Anteil von bis zu 10 Vol.-% zu der wirkstoffhaltigen Beschichtungslösung von Schritt c), bezogen auf das Gesamtvolumen von Wasser und organischem Lösungsmittel; und
e) Auftragen der wirkstoffhaltigen Beschichtungslösung von Schritt c) oder d) auf eine Oberfläche eines Ballonkatheters und Eintrocknen der Beschichtungslösung unter Bildung des beschichteten Ballonkatheters.

### BESCHREIBUNG BEVORZUGTER AUSFÜHRUNGSFORMEN

Als pharmazeutisch aktiver Wirkstoff wird im Sinne der Erfindung eine pharmakologisch wirksame Substanz verstanden, insbesondere ein Arzneistoff. Der pharmazeutisch aktive Wirkstoff ist vorzugsweise eine anti-proliferative, immunsuppressive, anti-inflammatorische, anti-phlogistische, anti-hyperplastische, anti-neoplastische, anti-mitotische, zytostatische, zytotoxische, anti-angiogene, antirestenotische, mikrotubuli-inhibierende, antimigrative oder antithrombotische Substanz. Bevorzugt ist ein anti-proliferativer Wirkstoff, insbesondere Paclitaxel und/oder Rapamycin, ein immunsuppressiver Wirkstoff wie Everolimus, Biolimus und/oder Tracrolimus, Kortison oder eine Kombination davon.

Der pharmazeutisch aktive Wirkstoff ist vorzugsweise löslich in polaren organischen Lösungsmitteln wie Methanol, Ethanol, Aceton, Ethylacetat, Trichlormethan und DMSO, jedoch unlöslich oder nur schwerlöslich in Wasser.

Gemäß einer bevorzugten Ausführungsform ist das PEG-PVA-Copolymer ein Propfcopolymer, besonders bevorzugt ein PEG-PVA-Propfcopolymer mit einem PEG-Anteil von 15 bis 30 Mol-% und einem PVA-Anteil von 70 bis 85 Mol-%. Derartige Pfropfcopolymere sind im Handel erhältlich und als Arzneimittelzusatzstoffe zugelassen. Nach Herstellerangaben ist das PEG-PVA-Copolymer löslich in Wasser sowie wässrigen Systemen wie schwachen Säuren oder Laugen. In einem Gemisch aus Wasser und Ethanol im Verhältnis 1:1 (v/v) können noch bis zu 25 Gewichts-% des PEG-PVA-Copolymers gelöst werden. Die handelsüblichen Erzeugnisse können Zusatzstoffe wie kolloidales Siliziumdioxid in geringen Mengen (unter 1 Gewichtsprozent) enthalten.

Bevorzugt weist das PEG-PVA-Copolymer eine mittlere Molmasse Mₙ von 30.000 bis 60.000 g/mol auf, bevorzugt von 40.000 bis 50.000 g/mol.

Gemäß einer weiteren Ausführungsform enthält die Bindermatrix ferner Schellack. Schellack begünstigt die Freisetzung des Wirkstoffs in das Gewebe der Blutgefäße, insbesondere bei kurzen Kontaktzeiten, und verbessert die Haftfestigkeit der Beschichtung auf dem Ballon.

Zusätzlich zu Schellack kann die Beschichtung weitere pharmazeutisch verträgliche Hilfsstoffe enthalten, wie sie dem Fachmann aus dem Stand der Technik bekannt sind.

Gemäß einer besonders bevorzugten Ausführungsform besteht die Bindermatrix aus dem PEG-PVA-Copolymer. In diesem Fall enthält die Bindermatrix keine weiteren Hilfsstoffe, außer den bereits in den Ausgangskomponenten der Bindermatrix enthaltenen Zusatzstoffe, die als unvermeidbare Verunreinigungen angesehen werden können.

Das Gewichtsverhältnis von Schellack zu PEG-PVA-Copolymer in der Beschichtung beträgt höchstens 1 : 1, vorzugsweise höchstens 1 : 2.

Die Beladung des Kathetersubstrats mit dem Wirkstoff beträgt vorzugsweise von 0,5 µg/mm² bis 10 µg/mm², bezogen auf die Außenfläche des Ballons in expandiertem Zustand, bevorzugt von 0,5 bis 5 µg/mm² und besonders bevorzugt von 1 bis 3 µg/mm².

Das Gewichtsverhältnis von Wirkstoff zu PEG-PVA-Copolymer in der Beschichtung beträgt vorzugsweise 10:1 bis 1:2, bevorzugt 5:1 bis 1:2, weiter bevorzugt 3:1 bis 1:2 oder 2:1 bis 1:2 und besonders bevorzugt 2:1 bis 1:1. Falls der Anteil des PEG-PVA-Copolymers zu niedrig ist, kann die Haftung der Beschichtung auf der Ballonoberfläche beeinträchtigt sein. Bei einem zu hohen Anteil des PEG-PVA-Copolymers in der Bindermatrix steigt die die Wasserlöslichkeit der Beschichtung und die Beständigkeit gegen eine Ablösung der Beschichtung von der Ballonoberfläche nimmt ab, während der Katheter durch die Blutgefäße transportiert wird.

Das gesamte Auftragsgewicht der Beschichtung auf der Ballonoberfläche liegt vorzugsweise in einem Bereich von 0,75 µg/mm² bis 20 µg/mm².

Bevorzugt liegt der Wirkstoff in Form von in die Beschichtung eingebetteten Wirkstoffteilchen vor, weiter bevorzugt in Form von nanoskopischen und/oder mikroskopschen Teilchen, und besonders bevorzugt in Form von sphäroidischen und/oder nadelförmigen Aggregaten, die in die Bindermatrix eingebettet sind. Die Bildung derartiger Aggregate deutet darauf hin, dass sich die Phasen von Wirkstoff und Bindermatrix wenigstens teilweise getrennt haben und bei geeigneten Bedingungen auch bereits mikroskopische Kristalle des Wirkstoffes entstanden sind. Eine solche Beschichtung mit in die Bindermatrix eingebetteten Wirkstoffteilchen ist unter den Bedingungen beim Transport des Katheters im Blutserum ausreichend stabil. Außerdem kann die Freisetzungsrate des Wirkstoffs am Stenoseort über die nanoskopische oder mikroskopische Struktur der Wirkstoffteilchen je nach Anwendungsfall gezielt gesteuert werden. Die Erfinder gehen davon aus, dass der in mikroskopischer Form auf das Gewebe übertragene Wirkstoff eine geringere Auflösungsgeschwindigkeit aufweist und dadurch länger am Stenoseort verbleiben kann, während der in Form von nanoskopischen Teilchen vorliegende Wirkstoff eine höhere Auflösungsgeschwindigkeit zeigt und dadurch eine hohe Anfangskonzentration des Wirkstoffs erreicht werden kann.

Nanoskopische oder nanoskalige Teilchen sind solche mit Abmessungen von 1 nm bis 100 nm. Als mikroskopische oder mikroskalige Körper werden Wirkstoffteilchen mit Abmessungen ab 0,1 µm bis etwa 100 µm angesehen.

Die Schicht mit dem in der Bindermatrix eingebetteten Wirkstoff ist bevorzugt die äußerste Schicht der Beschichtung auf dem Kathetersubstrat und besonders bevorzugt die einzige Schicht der Beschichtung. Gemäß einer alternativen Ausführungsform kann vorgesehen sein, eine wirkstofffreie Haftschicht auf dem Kathetersubstrat aufzubringen, auf der dann die wirkstoffhaltige Schicht mit PEG-PVA-Copolymer als Bindermatrix liegt. Das Auftragen einer Deckschicht auf der wirkstoffhaltigen Schicht ist erfindungsgemäß nicht vorgesehen.

Als Kathetersubstrat können alle handelsüblichen Ballone von Ballonkathetern verwendet werden. Insbesondere eignen sich Ballone mit glatter Oberfläche, Ballone mit Furchen oder Poren und Ballone mit strukturierten oder aufgerauten Oberflächen. Geeignet sind weiter Ballone von Kathetern, die mit Falten oder Flügeln versehen sind, insbesondere sogenannte Mehrfaltenballone.

Die Herstellung des beschichteten Ballonkatheters erfolgt durch Auftragen einer Beschichtungslösung auf eine Oberfläche des Kathetersubstrats, welche alle festen Komponenten der Beschichtung enthält. Das Auftragen der Beschichtungslösung kann durch Sprühbeschichten, Tauchbeschichten oder Druckverfahren erfolgen, wie sie dem Fachmann bekannt sind.

Gegenstand der Erfindung ist daher ferner eine Zusammensetzung zur Beschichtung eines Ballonkatheters, die einen pharmazeutisch aktiven Wirkstoff, insbesondere einen anti-inflammatorischen Wirkstoff wie Kortison, einen immunosuppressiven Wirkstoff wie Everolimus, Biolimus und/oder Tracrolimus oder einen anti-proliferativen Wirkstoff wie Paclitaxel und/oder Rapamycin und ein Bindemittel umfasst, das aus einem PEG-PVA-Copolymer und wahlweise Schellack besteht. Der Wirkstoff und das Bindemittel sind in einem Lösungsmittelgemisch gelöst, das aus Wasser und wenigstens einem weiteren organischen Lösungsmittel besteht, das mit Wasser unbegrenzt mischbar ist und wahlweise bis zu 10 Vol.-% DMSO enthält.

Als pharmazeutischer Wirkstoff können die schon beschriebenen Wirkstoffe verwendet werden. Das PEG-PVA-Copolymer ist bevorzugt ein PEG-PVA-Propfcopolymer, wie oben beschrieben. PEG-PVA-Copolymere für pharmazeutische und kosmetische Anwendungen sind im Handel erhältlich.

Gemäß einer bevorzugten Ausführungsform enthält die Zusammensetzung bis zu etwa 5 bis 40 Vol.-% Wasser, bevorzugt von etwa 5 Vol.-% bis 35 Vol.-%, weiter bevorzugt von 10-35 Vol.-% und ganz besonders bevorzugt von 15-25 Vol.-% Wasser, bezogen auf den Anteil des Gemischs aus Wasser und weiterem organischem Lösungsmittel.

Das weitere organische Lösungsmittel weist vorzugweise einem Siedepunkt von unter 100 °C auf. Besonders bevorzugt sind Aceton sowie niedere Alkohole wie Methanol, Ethanol und/oder Isopropanol, besonders bevorzugt Ethanol. Bevorzugt enthält die Zusammensetzung etwa 60 bis 95 Vol.-%, bevorzugt von etwa 65 Vol.-% bis 95 Vol.-%, weiter bevorzugt von 65 bis 90 Vol.-% und ganz besonders bevorzugt von 75 bis 85 Vol.-% des weiteren organischen Lösungsmittels, bezogen auf den Anteil des Gemischs aus Wasser und weiterem organischem Lösungsmittel.

Weiter bevorzugt ist in der Zusammensetzung DMSO in einem Anteil von additiv 0,5-10 Vol.-% enthalten, bevorzugt bis zu 5 Vol.-% und besonders bevorzugt 2 bis 4 Vol.-%, bezogen auf das Volumen des Gemischs aus Wasser und weiterem organischem Lösungsmittel.

Der pharmazeutisch aktive Wirkstoff kann in einer Konzentration von bis zu 25 mg/ml in der Zusammensetzung vorliegen. Bevorzugt sind Wirkstoffkonzentrationen von bis zu 20 mg oder 15 mg pro 1 ml Lösung, mehr bevorzugt bis zu 10 mg Wirkstoff pro 1 ml Lösung, insbesondere 0,5 mg bis 20 mg, bevorzugt, 1 bis 15 mg oder 5 mg bis 15 mg Wirkstoff pro 1 ml Lösung.

Das Gewichtsverhältnis des Wirkstoffs zu PEG-PVA-Copolymer als Bindemittel beträgt vorzugsweise 10:1 bis 1:2, bevorzugt 5:1 bis 1:2, weiter bevorzugt 3:1 bis 1:2 oder 2:1 bis 1:2 und besonders bevorzugt 2:1 bis 1:1. Falls Schellack als weiteres Bindemittel eingesetzt wird, beträgt das Gewichtsverhältnis von Schellack zu dem PEG-PVA-Copolymer bevorzugt höchstens 1:1, besonders bevorzugt höchstens 1:2.

Der Gesamtfeststoffanteil der Zusammensetzung kann bis zu 150 mg/ml Lösung betragen, bevorzugt bis zu 125 mg/ml und besonders bevorzugt bis zu 100 mg/ml. Besonders bevorzugt ist ein Gesamtfeststoffanteil von 1 mg bis 100 mg pro 1 ml Lösung, mehr bevorzugt zwischen 1 mg bis 50 mg pro 1 ml Lösung und am meisten bevorzugt von 1 mg bis 40 mg oder 1 bis 30 mg pro 1 ml Lösung.

Gemäß einer besonderen Ausführungsform umfasst die Zusammensetzung einen anti-proliferativen und/oder immunsuppressiven Wirkstoff, insbesondere Paclitaxel, Rapamycin, Everolimus, Biolimus und/oder Tacrolimus in einer Konzentration von bis zu 25 mg/ml, sowie ein PEG-PVA-Copolymer in einer Konzentration von 1 bis 50 mg/ml. Das Lösungsmittel für die Feststoffe besteht aus einem Gemisch von Wasser, Ethanol und/oder Methanol sowie wahlweise DMSO. Das Wasser liegt vorzugsweise in einen Anteil von 5 bis 40 Vol.-%, bevorzugt 5-35 Vol.-%, vor, bezogen auf das Gesamtvolumen von Wasser sowie Ethanol und/oder Methanol. Das DMSO kann in einem Anteil von 0,5 bis 10 Vol.-%, bevorzugt bis 1 bis 5 Vol.-%, vorzugweise 2 bis 4 Vol.-%, zugesetzt werden, bezogen auf das Gesamtvolumen von Wasser sowie Ethanol und/oder Methanol.

Zur Herstellung der Beschichtungslösung wird das PEG-PVA-Copolymer in Wasser gelöst und schrittweise mit dem weiteren organischen Lösungsmittel, beispielsweise Ethanol, versetzt. Anschließend wird der pharmazeutisch aktive Wirkstoff zu der wässrig-organischen Lösung des Bindemittels zugesetzt, vorzugsweise als Feststoff, und das Gemisch wird homogenisiert. Zu der so erhaltenen Lösung kann anschließend DMSO in einem Anteil von bis zu 10 Vol.-% zugesetzt, werden bezogen auf das Gesamtvolumen von Wasser und weiterem Lösungsmittel. Der Schellack kann wahlweise auch zur wässrig-ethanolischen Lösung des PEG-PVA-Copolymers, vor oder nach der Zugabe des Wirkstoffs, zugegeben werden.

Überraschenderweise verbleibt das PEG-PVA-Copolymer bei schrittweiser Zugabe des weiteren organischen Lösungsmittels, insbesondere Ethanol, in Lösung und bildet eine stabile, wässrig-ethanolische Polymerlösung, in welcher der Wirkstoff homogen gelöst werden kann.

Das Auftragen der Beschichtungslösung auf die Oberfläche des Kathetersubstrats kann im nicht expandierten Zustand oder in einem ganz oder teilweise expandierten Zustand des Ballons erfolgen. Insbesondere mit Falten versehene Ballone werden vorzugsweise in einem wenigstens teilweise expandierten Zustand beschichtet.

Zur Verbesserung der Benetzung mit Beschichtungslösung kann die Ballonoberfläche vor der Beschichtung einer Koronabehandlung oder einer Plasmabehandlung unterzogen werden.

Nach dem Auftragen der Beschichtungslösung wird die Ballonoberfläche vorzugweise in Warmluft und/oder unter Vakuum getrocknet, so dass im Wesentlichen kein Lösungsmittel in der Beschichtung verbleibt. Soweit flüssige oder gelbildende Hilfsstoffe verwendet werden, die pharmazeutisch verträglich sind, können diese in der Beschichtung verbleiben.

Schließlich kann der beschichtete Ballon mit bekannten Maßnahmen, beispielsweise durch Behandeln mit Ethylenoxid, sterilisiert und steril verpackt werden.

Durch das Auftragen der Beschichtungslösung auf das Substrat wird ein homogener dünner Flüssigkeitsfilm erzeugt, der die festen Komponenten der Beschichtungslösung und wenigstens zwei verschiedene Lösungsmittel mit unterschiedlichen Dampfdrücken enthält, bevorzugt wenigstens drei verschiedene Lösungsmittel. Beim Trocknen des beschichteten Ballons reichert sich Wasser in der Beschichtungslösung auf der Ballonoberfläche an, da zunächst das niedriger siedende organische Lösungsmittel wahlweise als Azeotrop mit Wasser verdampft. Der in Wasser nicht lösliche Wirkstoff, bevorzugt das Paclitaxel, fällt unter Bildung von nanoskopischen Teilchen aus der Lösung aus, die in getrocknetem Bindemittel eingebettet sind. Dieser Schritt ist an der Bildung einer weißlich erscheinenden Schicht zu erkennen.

Sobald die Löslichkeitsgrenze des Bindemittels in Wasser überschritten wird, trocknet auch die Bindermatrix an. Die Beschichtung erscheint trocken und es bildet sich eine zähe, klare bis trübe Schicht aus dem Bindemittel, die den von der Binderphase getrennten Wirkstoff einhüllt und fest mit der Ballonoberfläche verbindet. Im Vergleich zur Trocknung aus einem wasserfreien Lösungsmittel verläuft die Phasentrennung der Bindermatrix und des Wirkstoffs jedoch über einen längeren Zeitraum, so dass die Bildung nanoskopischer Strukturen, meist in Form kugelförmiger oder nadelförmiger Aggregate des Wirkstoffs, begünstigt ist.

Die Erfinder haben ferner erkannt, dass das als Bindemittel verwendete PEG-PVA-Copolymer ebenfalls in DMSO löslich ist. Die Zugabe einer geringen Menge von DMSO zur Beschichtungslösung führt somit dazu, dass das in der Beschichtung noch enthaltene DMSO sowohl den Wirkstoff, insbesondere Paclitaxel, als auch die Bindermatrix aus PEG-PVA-Copolymer und wahlweise Schellack lösen kann. Je nach Anteil und Zusammensetzung der Bindermatrix und der Lösungsmittel in der Beschichtungslösung kann sich deshalb der Wirkstoff während des Trocknungsprozesses aus der Bindermatrix in vielfältiger Form ausscheiden und eine heterogene, haftfeste Beschichtung mit von der Bindermatrix getrennten nanoskopischen und/oder mikroskopischen Wirkstoffteilchen bilden. Über die Dauer des Trocknungsprozesses kann die Rekristallisation der Bindermatrix und des Wirkstoffs weiter gesteuert werden.

Enthält die Beschichtungslösung kein DMSO, entstehen nach dem oben beschriebenen Prozess überwiegend nanoskopische Wirkstoffteilchen, die getrennt von der Bindermatrix vorliegen und in diese eingebettet sind. Ein Zusatz von DMSO zur Beschichtungslösung begünstigt das Entstehen mikroskopischer Strukturen der Wirkstoffteilchen.

Ist der Anteil an DMSO in der Beschichtungslösung so bemessen, dass die während des Abtrockens der Lösungsmittel in der Beschichtung vorliegende Menge an DMSO nicht dazu ausreicht, die Bindermatrix und das Paclitaxel vollständig zu lösen, wird der Trocknungsprozess verlängert und das Paclitaxel kann in eine mikroskopische Stäbchen- oder Nadelstruktur übergehen, die nur schwer in Wasser löslich ist. Während der sehr langsamen Abtrocknung des DMSO aus der Beschichtung wird die Binderphase somit von der mikroskopischen Paclitaxelphase getrennt. Die Wasserlöslichkeit der Beschichtung wird dann in erster Linie von dem Gewichtsverhältnis der Anteile des wasserlöslichen Bindemittels und des in Wasser schwer löslichen Wirkstoffs in der Beschichtung beeinflusst.

Ist der Anteil an DMSO in der Beschichtungslösung dagegen so hoch, dass die in der Beschichtung verbleibende Menge an DMSO sowohl das Paclitaxel als auch das Bindemittel lösen kann, entsteht eine unstrukturierte klare Beschichtung. Der wahlweise Zusatz und/oder die Optimierung des DMSO-Anteils in der Beschichtungslösung ermöglicht daher die gezielte Ausbildung von schwerlöslichen, nanoskaligen und/oder mikroskaligen Wirkstoffstrukturen in der Beschichtung.

Unter Verwendung der erfindungsgemäßen Beschichtungslösung lässt sich ein beschichteter Ballonkatheter mit einer festhaftenden Beschichtung aus einem anti-proliferativen Wirkstoff wie Paclitaxel und einer mechanisch sehr stabilen Bindermatrix aus PEG-PVA-Copolymer und wahlweise Schellack oder einem Schellackderivat herstellen, aus der das Paclitaxel je nach Anwendungsfall in Form von nanoskopischen und/oder mikroskopischen Teilchen gezielt am Stenoseort durch Deflation des Ballons in die Gefäßwand übertragen und von dort durch Diffusion in das Gewebe des Gefäßes freigesetzt werden kann. Die Dauer der Freisetzung und damit die zytostatische Wirkung des Paclitaxels wächst mit der Größe und der Kristallinität der Wirkstoffteilchen und kann damit insbesondere durch eine entsprechende Änderung der Zusammensetzung der Beschichtungslösung beeinflusst werden.

Weitere Merkmale und Vorteile der Erfindung ergeben sich aus der nachfolgenden Beschreibung eines bevorzugten Ausführungsbeispiels unter Bezug auf die beigefügte Zeichnung. Das Ausführungsbeispiel soll jedoch nicht in einem einschränkenden Sinn verstanden werden.

### BESCHREIBUNG DER ZEICHNUNGEN

- Figur 1 zeigt eine elektronenmikroskopische Aufnahme einer Paclitaxel/PEG-PVA/Schellack-Beschichtung in der Zusammensetzung 2:1:0,5 in einer 5000-fachen Vergrößerung, erhalten aus einer Beschichtungslösung ohne DMSO; und
- Figur 2 zeigt eine elektronenmikroskopische Aufnahme einer Paclitaxel/PEG-PVA-Beschichtung in einer 5000-fachen Vergrößerung, die ein Verhältnis von PEG-PVA zu Paclitaxel von 1:2 aufweist, erhalten aus einer Beschichtungslösung, die zusätzlich 3 % DMSO enthält.

### HERSTELLUNG EINER BESCHICHTUNGSLÖSUNG

### Beispiel 1 (Referenzbeispiel)

Zunächst wurden 25 mg eines PEG-PVA-Propfcopolymers in 2 ml Wasser gelöst. Der Anteil von PEG im PEG-PVA-Propfcopolymer betrug 25 mol-%. Durch feindosierte, schrittweise Zugabe von 8 ml Ethanol ergab sich eine homogene Polymerlösung. Der antiproliferative Wirkstoff Paclitaxel wurde in einer Menge von 50 mg als Feststoff bereitgestellt und unter Einwirkung von Ultraschall der Lösung beigefügt. Anschließend wurde der Lösung ein Anteil von 12,5 mg eines Ammoniumsalzes von Schellack zugesetzt. Man erhielt eine klare Lösung mit einem Feststoffanteil von 8,75 mg/ml Lösungsmittel bei einem Volumenverhältnis Ethanol:Wasser von 80:20.

### Beispiel 2 (erfindungsgemäß)

Wie in Beispiel 1 beschrieben, wurden 25 mg eines PEG-PVA-Propfcopolymers in 2 ml Wasser gelöst. Der Anteil von PEG im PEG-PVA-Propfcopolymer betrug 25 mol-%. Durch feindosierte, schrittweise Zugabe von 8 ml Ethanol ergab sich eine homogene Polymerlösung. Der antiproliferative Wirkstoff Paclitaxel wurde in einer Menge von 50 mg als Feststoff bereitgestellt und unter Einwirkung von Ultraschall der Lösung beigefügt. Zu dieser Lösung wurden zusätzlich 0,3 ml DMSO zugesetzt. Man erhielt eine klare Lösung mit einem Feststoffanteil von 7,5 mg/ml Lösungsmittel bei einem Volumenverhältnis Ethanol: Wasser von 80:20 und einem DMSO-Anteil von additiv 3 %, bezogen auf das Gesamtvolumen von Ethanol und Wasser. Durch Lösungsversuche wurde festgestellt, dass der Wasseranteil der Beschichtungslösung auf bis zu 40 Vol.-% erhöht werden kann, ohne dass das Paclitaxel aus der Lösung ausfällt.

### Beispiel 3

Wie im Beispiel 2 beschrieben, wurde eine Lösung von 25 mg eines PEG-PVA-Propfcopolymers in 2 ml Wasser hergestellt. Der Anteil von PEG im PEG-PVA-Propfcopolymer betrug 25 mol-%. Durch feindosierte schrittweise Zugabe von 8 ml Ethanol wurde eine homogene Polymerlösung erhalten. Der antiproliferative Wirkstoff Paclitaxel wurde in einer Menge von 50 mg als Feststoff bereitgestellt und unter Einwirkung von Ultraschall der Lösung beigefügt. Man erhielt eine stabile, klare Lösung mit einem Feststoffanteil von 7,5 mg/ml Lösungsmittel bei einem Volumenverhältnis Ethanol:Wasser von 80:20. Die Lösung enthielt kein DMSO.

### BESCHICHTUNGSVERSUCHE

Mit der Beschichtungslösung gemäß Beispiel 1 wurde ein glattwandiger Ballon eines handelsüblichen Ballonkatheters beschichtet. Der Auftrag der Beschichtungslösung auf die Ballonoberfläche des Katheters erfolgte durch Sprühbeschichten.

Nach dem Auftragen der Beschichtungslösung wurde die Ballonoberfläche mit Warmluft bei einer Temperatur von zwischen 30 und 60 °C getrocknet. Nach der Trocknung blieb eine festhaftende und mechanisch stabile Beschichtung aus im Wesentlichen nanoskopischem Paclitaxel in Form von nadelförmigen Teilchen zurück, die in eine Bindermatrix aus dem PEG-PVA-Copolymer und Schellack eingebettet waren.

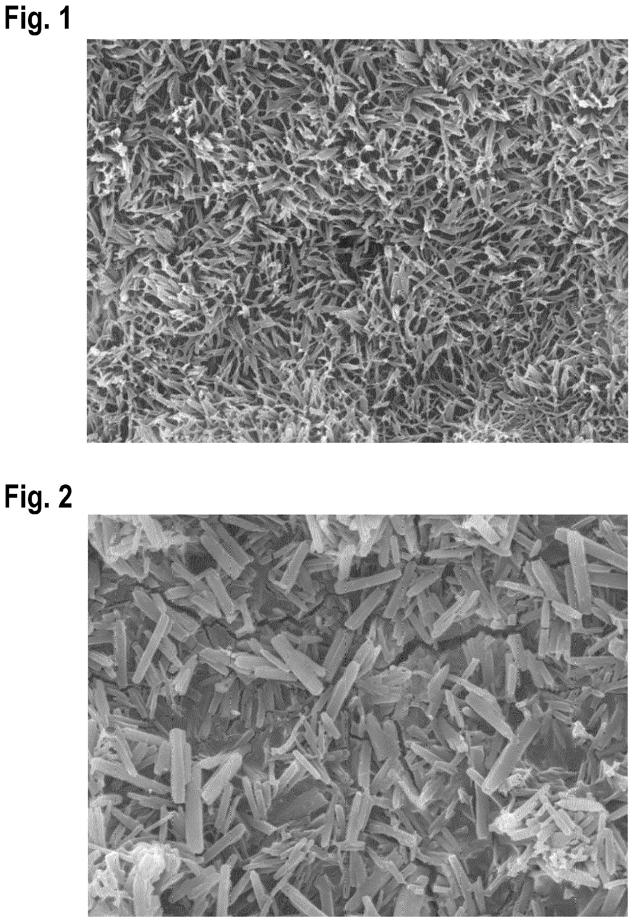

In der in Fig. 1 gezeigten elektronenmikroskopischen Aufnahme einer unter Verwendung der erfindungsgemäßen Beschichtungslösung gemäß Beispiel 1 hergestellten Beschichtung ist zu erkennen, dass die Beschichtung in Form von nadelförmigen nanoskopischen Teilchen aus Paclitaxel vorliegt, die wie in einem Netzwerk miteinander verbunden sind .

Die Beschichtungslösung gemäß Beispiel 2, ohne Schellackzusatz jedoch mit einem Anteil von additiv 3 % DMSO, ergab nach dem Trockenen auf der Ballonoberfläche ebenfalls eine stabile und festhaftende Beschichtung aus nadelförmigen Paclitaxel-Mikroteilchen in einer Bindermatrix aus PEG-PVA-Copolymer.

Fig. 2 zeigt eine elektronenmikroskopische Aufnahme der mit der Beschichtungslösung gemäß Beispiel 2 erhaltenen Beschichtung, die die DMSO aber keinen Schellack enthielt. Die Beschichtung ist mikroskopisch nadelförmig strukturiert und zeigt eine deutliche Phasentrennung.

Die Beschichtungslösung gemäß Beispiel 3 ergab eine Beschichtung mit einer nanoskopischen Struktur der Wirkstoffteilchen, ähnlich der mit der Beschichtungslösung von Beispiel 1 erhaltenen Beschichtung.

Die Beladung der Ballonoberfläche des Katheters mit Paclitaxel kann mit dem erfindungsgemäßen Verfahren frei eingestellt werden. Über das Verhältnis von Wirkstoff zu wasserlöslichem Bindemittel kann sowohl die Beständigkeit der Beschichtung als auch die Strukturierung des Wirkstoffs weiter gesteuert werden.

Der so hergestellte Ballonkatheter eignet sich insbesondere zur Behandlung von Restenosen nach einer Gefäßdilatation oder einer Stenteinsetzung.

## Patentansprüche

1. Beschichteter Ballonkatheter mit einem Kathetersubstrat und einer Beschichtung auf dem Kathetersubstrat, wobei die Beschichtung eine äußerste Schicht mit einem in einer Bindermatrix eingebetteten pharmazeutisch aktiven Wirkstoff umfasst, **dadurch gekennzeichnet dass** die Bindermatrix aus einem Polyethylenglycol-Polyvinylalkohol-Copolymer (PEG-PVA-Copolymer) und wahlweise Schellack besteht.

2. Ballonkatheter nach Anspruch 1, **dadurch gekennzeichnet, dass** der Wirkstoff aus der aus anti-proliferativen, immunsuppressiven, anti-inflammatorischen, anti-phlogistischen, anti-hyperplastischen, anti-neoplastischen, anti-mitotischen, zytostatischen, zytotoxischen, anti-angiogenen, antirestenotischen, mikrotubuli-inhibierenden, antimigrativen und oder antithrombotischen Wirkstoffen bestehenden Gruppe ausgewählt ist, insbesondere Kortison, Everolimus, Biolimus, Tacrolimus, Paclitaxel und/oder Rapamycin ist.

3. Ballonkatheter nach Anspruch 1 oder 2, **dadurch gekennzeichnet, dass** das PEG-PVA-Copolymer ein Propfcopolymer ist, bevorzugt, dass das PEG-PVA-Copolymer einen PEG-Anteil von 15 bis 30 Mol-% und einen PVA-Anteil von 70 bis 85 Mol-% enthält.

4. Ballonkatheter nach einem der Ansprüche 1 bis 3, **dadurch gekennzeichnet, dass** das PEG-PVA-Copolymer eine mittlere Molmasse Mₙ von 30.000 bis 60.000 g/mol aufweist, bevorzugt von 40.000 bis 50.000 g/mol.

5. Ballonkatheter nach einem der Ansprüche 1 bis 4, **dadurch gekennzeichnet, dass** das Gewichtsverhältnis von Wirkstoff zu PEG-PVA-Copolymer in der Beschichtung in einem Bereich von 10:1 bis 1:2 liegt, bevorzugt von 2:1 bis 1:1.

6. Ballonkatheter nach einem der Ansprüche 1 bis 5, **dadurch gekennzeichnet, dass** die Bindermatrix Schellack enthält, wobei das Gewichtsverhältnis von Schellack zu PEG-PVA-Copolymer in der Beschichtung bevorzugt höchstens 1:1 beträgt, vorzugsweise höchstens 1:2.

7. Ballonkatheter nach einem der Ansprüche 1 bis 6, **dadurch gekennzeichnet, dass** die Beladung des Kathetersubstrats mit dem Wirkstoff von 0,5 µg/mm² bis 10 µg/mm² beträgt, bezogen auf eine Außenfläche des Substrats in expandiertem Zustand, bevorzugt von 0,5 bis 5 µg/mm², und besonders bevorzugt von 1 bis 3 µg/mm².

8. Ballonkatheter nach einem der Ansprüche 1 bis 7, **dadurch gekennzeichnet, dass** das Auftragsgewicht der Beschichtung auf das Kathetersubstrat von 0,75 µg/mm² bis 20 µg/mm² beträgt.

9. Ballonkatheter nach einem der Ansprüche 1 bis 8, **dadurch gekennzeichnet, dass** der Wirkstoff in Form von in die Bindermatrix eingebetteten Wirkstoffteilchen, bevorzugt in Form von nanoskopischen und/oder mikroskopischen Teilchen, vorliegt.

10. Zusammensetzung zur Beschichtung eines Ballonkatheters, wobei die Zusammensetzung einen pharmazeutisch aktiven Wirkstoff und ein Bindemittel umfasst, das aus einem PEG-PVA-Copolymer und wahlweise Schellack besteht, wobei der pharmazeutisch aktive Wirkstoff und das Bindemittel in einem Gemisch von Wasser und wenigstens einem weiteren mit Wasser unbegrenzt mischbaren organischen Lösungsmittel gelöst sind.

11. Zusammensetzung nach Anspruch 10, **dadurch gekennzeichnet, dass** die Zusammensetzung Wasser in einem Anteil von 5-40 Vol.-% enthält, und bevorzugt dass die Zusammensetzung zusätzlich DMSO in einem Anteil von bis zu 10 Vol.-% enthält, vorzugsweise 0,5 bis 10 Vol.-%, bezogen auf das Gesamtvolumen von Wasser und dem weiteren organischen Lösungsmittel.

12. Zusammensetzung nach Anspruch 10 oder 11, **dadurch gekennzeichnet, dass** das weitere organische Lösungsmittel Ethanol und/oder Methanol umfasst.

13. Zusammensetzung nach einem der Ansprüche 10 bis 12, **dadurch gekennzeichnet, dass** die Konzentration des Wirkstoffes in der Beschichtungslösung bis zu 25 mg/ml beträgt, und bevorzugt dass der Feststoffanteil in der Beschichtungslösung bis zu 150 mg/ml beträgt.

14. Zusammensetzung nach einem der Ansprüche 10 bis 13, bestehend aus einem antiproliferativen und/oder immunsuppressiven Wirkstoff, bevorzugt Paclitaxel, Rapamycin, Everolimus, Biolimus und/oder Tacrolimus, in einer Konzentration von bis zu 25 mg/ml, einem PEG-PVA-Copolymer in einer Konzentration von 1 bis 50 mg/ml, Wasser in einem Anteil von 5 bis 40 Vol.-%, Methanol und/oder Ethanol in einem Anteil von 60 bis 94 Vol.-% und DMSO in einem Anteil von 0 bis 10 Vol.-%, vorzugsweise 0,5 bis 10 Vol.-%.

15. Verwendung der Zusammensetzung gemäß einem der Ansprüche 10 bis 14 als Beschichtungslösung zur Herstellung eines beschichteten Ballonkatheters gemäß einem der Ansprüche 1 bis 9.

16. Verfahren zur Herstellung eines beschichteten Ballonkatheters gemäß einem der Ansprüche 1 bis 9, welches die folgenden Schritte umfasst:
a) Bereitstellen einer wässrigen Lösung eines PEG-PVA-Copolymers ;
b) schrittweise Versetzen der wässrigen Lösung von Schritt a) mit wenigstens einem weiteren, mit Wasser unbegrenzt mischbaren organischen Lösungsmittel unter Bildung einer wässrig-organischen Lösung;
c) Mischen der wässrig-organischen Lösung mit einem pharmazeutisch aktiven Wirkstoff und Homogenisieren des Gemischs unter Bildung einer wirkstoffhaltigen Beschichtungslösung;
d) wahlweise Zugeben von DMSO in einem Anteil von bis zu 10 Vol.-% zu der wirkstoffhaltigen Beschichtungslösung von Schritt c), bezogen auf das Gesamtvolumen von Wasser und organischem Lösungsmittel;
e) Auftragen der wirkstoffhaltigen Beschichtungslösung von Schritt c) oder d) auf eine Oberfläche eines Ballonkatheters und Eintrocknen der Beschichtungslösung unter Bildung des beschichteten Ballonkatheters.

## Claims

1. A coated balloon catheter comprising a catheter substrate and a coating on the catheter substrate, the coating comprising an outermost layer with a pharmaceutically active ingredient embedded in a binder matrix, **characterized in that** the binder matrix consists of a polyethylene glycol-polyvinyl alcohol copolymer (PEG-PVA copolymer) and optionally shellac.

2. The balloon catheter according to claim 1, **characterized in that** the active ingredient is selected from the group consisting of anti-proliferative, immunosuppressive, anti-inflammatory, anti-phlogistic, anti-hyperplastic, antineoplastic, anti-mitotic, cytostatic, cytotoxic, anti-angiogenic, anti-restenotic, microtubule inhibiting, anti-migrative and/or anti-thrombotic active ingredients, in particular is cortisone, everolimus, biolimus, tacrolimus, paclitaxel and/or rapamycin.

3. The balloon catheter according to claim 1 or 2, **characterized in that** the PEG-PVA copolymer is a graft copolymer, preferably that the PEG-PVA copolymer has a PEG content of from 15 to 30 mole percent and a PVA content of from 70 to 85 mole percent.

4. The balloon catheter according to any of claims 1 to 3, **characterized in that** the PEG-PVA copolymer has an average molar mass Mₙ of from 30,000 to 60,000 g/mole, preferably from 40,000 to 50,000 g/mole.

5. The balloon catheter according to any of claims 1 to 4, **characterized in that** the weight ratio of the active ingredient to the PEG-PVA copolymer in the coating is in a range of from 10:1 to 1:2, preferably from 2:1 to 1:1.

6. The balloon catheter according to any of claims 1 to 5, **characterized in that** the binder matrix contains shellac, the weight ratio of shellac to the PEG-PVA copolymer in the coating is preferred to be at most 1:1, preferably at most 1:2.

7. The balloon catheter according to any of claims 1 to 6, **characterized in that** the loading of the catheter substrate with the active ingredient is in a range of from 0.5 µg/mm² to 10 µg/mm², based on an outer surface of the substrate in the expanded state, preferably from 0.5 to 5 µg/mm² and particularly preferably from 1 to 3 µg/mm².

8. The balloon catheter according to any of claims 1 to 7, **characterized in that** the weight of the coating applied onto the catheter substrate is in a range of from 0.75 µg/mm² to 20 µg/mm².

9. The balloon catheter according to any of claims 1 to 8, **characterized in that** the active ingredient is present in the form of active ingredient particles embedded in the binder matrix, preferably in the form of nanoscopic and/or microscopic particles.

10. A composition for coating a balloon catheter, the composition comprising a pharmaceutically active ingredient and a binder which consists of a PEG-PVA copolymer and optionally shellac, the pharmaceutically active ingredient and the binder being dissolved in a mixture of water and at least one additional organic solvent indefinitely miscible with water.

11. The composition according to claim 10, **characterized in that** the composition contains water in a percentage of 5 to 40 volume percent, and preferably that the composition additionally contains DMSO in a percentage of up to 10 volume percent, preferably 0.5 to 10 volume percent, based on the total volume of water and the additional organic solvent.

12. The composition according to claim 10 or 11, **characterized in that** the additional organic solvent comprises ethanol and/or methanol.

13. The composition according to any of claims 10 to 12, **characterized in that** the concentration of the active ingredient in the coating solution is up to 25 mg/ml, and preferably that the solids content in the coating solution is up to 150 mg/ml.

14. The composition according to any of claims 10 to 13, consisting of an anti-proliferative and/or immunosuppressive active ingredient, preferably paclitaxel, rapamycin, everolimus, biolimus and/or tacrolimus, at a concentration of up to 25 mg/ml, a PEG-PVA copolymer at a concentration of from 1 to 50 mg/ml, water in an amount of from 5 to 40 volume percent, methanol and/or ethanol in an amount of from 60 to 94 volume percent, and DMSO in a percentage of from 0 to 10 volume percent, preferably 0.5 to 10 volume percent.

15. Use of the composition according to any of claims 10 to 14 as a coating solution for the production of a coated balloon catheter according to any of claims 1 to 9.

16. A method for the production of a coated balloon catheter according to any of claims 1 to 9, comprising the following steps:
a) providing an aqueous solution of a PEG-PVA copolymer;
b) gradually adding at least one additional organic solvent indefinitely miscible with water to the aqueous solution of step a) to form an aqueous-organic solution;
c) mixing the aqueous-organic solution with a pharmaceutically active ingredient and homogenizing the mixture to form a coating solution containing the active ingredient;
d) optionally adding DMSO in a percentage of up to 10 volume percent to the coating solution containing the active ingredient of step c), based on the total volume of water and organic solvent;
e) applying the coating solution containing the active ingredient of step c) or d) onto a surface of a balloon catheter and drying the coating solution to form the coated balloon catheter.

## Revendications

1. Cathéter à ballonnet revêtu, présentant un substrat de cathéter et un revêtement sur le substrat de cathéter, le revêtement comprenant une couche extérieure qui contient un principe pharmaceutiquement actif noyé dans une matrice liante, **caractérisé en ce que** la matrice liante est composée d'un copolymère de polyéthylène glycol et d'alcool polyvinylique (copolymère de PEG et de PVA) et optionnellement de gomme-laque.

2. Cathéter à ballonnet selon la revendication 1, **caractérisé en ce que** le principe actif est choisi dans le groupe constitué de principes actifs antiprolifératifs, immunosuppresseurs, antiinflammatoires, antiphlogistiques, antihyperplasiques, antinéoplasiques, antimitotiques, cytostatiques, cytotoxiques, antiangiogéniques, antiresténotiques, inhibiteur de microtubules, antimigratoires et/ou antithrombotiques, et est en particulier de la cortisone, de l'évérolimus, du biolimus, du tacrolimus, du paclitaxel et/ou de la rapamycine.

3. Cathéter à ballonnet selon la revendication 1 ou 2, **caractérisé en ce que** le copolymère de PEG et de PVA est un copolymère greffé, de préférence **en ce que** le copolymère de PEG et de PVA contient une quantité de PEG de 15 à 30 % mol et une quantité de PVA de 70 à 85 % mol.

4. Cathéter à ballonnet selon l'une des revendications 1 à 4, **caractérisé en ce que** le copolymère de PEG et de PVA présente une masse molaire moyenne Mₙ de 30.000 à 60.000 g/mol, de préférence de 40.000 à 50.000 g/mol.

5. Cathéter à ballonnet selon l'une des revendications 1 à 4, **caractérisé en ce que** le rapport pondéral du principe actif au copolymère de PEG et de PVA dans le revêtement est dans une plage de 10:1 à 1:2, de préférence de 2:1 à 1:1.

6. Cathéter à ballonnet selon l'une des revendications 1 à 5, **caractérisé en ce que** la matrice liante contient de la gomme-laque, le rapport pondéral de la gomme-laque au copolymère de PEG et de PVA dans le revêtement étant de préférence de 1:1 au maximum, de préférence de 1:2 au maximum.

7. Cathéter à ballonnet selon l'une des revendications 1 à 6, **caractérisé en ce que** le chargement du substrat de cathéter avec le principe actif est de 0,5 µg/mm² à 10 µg/mm² par rapport à une surface extérieure du substrat à l'état expansé, de préférence de 0,5 à 5 µg/mm², et de manière particulièrement préférée de 1 à 3 µg/mm².

8. Cathéter à ballonnet selon l'une des revendications 1 à 7, **caractérisé en ce que** le poids d'application du revêtement sur le substrat de cathéter est de 0,75 µg/mm² à 20 µg/mm².

9. Cathéter à ballonnet selon l'une des revendications 1 à 8, **caractérisé en ce que** le principe actif est présent sous forme de particules de principe actif noyées dans la matrice liante, de préférence sous forme de particules nanoscopiques et/ou microscopiques.

10. Composition pour le revêtement d'un cathéter à ballonnet, la composition comprenant un principe pharmaceutiquement actif et un liant composé d'un copolymère de PEG et de PVA et optionnellement de gomme-laque, le principe pharmaceutiquement actif et le liant étant dissous dans un mélange d'eau et d'au moins un solvant organique supplémentaire miscible sans limites avec de l'eau.

11. Composition selon la revendication 10, **caractérisée en ce que** la composition contient de l'eau dans une quantité de 5-40 % en volume, et de préférence **en ce que** la composition contient en plus du DMSO dans une quantité allant jusqu'à 10 % en volume, de préférence de 0,5 à 10 % en volume par rapport au volume total de l'eau et du solvant organique supplémentaire.

12. Composition selon la revendication 10 ou 11, **caractérisée en ce que** le solvant organique supplémentaire comprend de l'éthanol et/ou du méthanol.

13. Composition selon l'une des revendications 10 à 12, **caractérisée en ce que** la concentration du principe actif dans la solution de revêtement s'élève jusqu'à 25 mg/ml, et de préférence **en ce que** la quantité de matières solides dans la solution de revêtement s'élève jusqu'à 150 mg/ml.

14. Composition selon l'une des revendications 10 à 13, composée d'un principe actif antiprolifératif et/ou immunosuppresseur, de préférence de paclitaxel, de rapamycine, d'évérolimus, de biolimus et/ou de tacrolimus dans une concentration allant jusqu'à 25 mg/ml, d'un copolymère de PEG et de PVA dans une concentration de 1 à 50 mg/ml, d'eau dans une quantité de 5 à 40 % en volume, de méthanol et/ou d'éthanol dans une quantité de 60 à 94 % en volume, et de DMSO dans une quantité de 0 à 10 % en volume, de préférence de 0,5 à 10 % en volume.

15. Utilisation de la composition selon l'une des revendications 10 à 14 en tant que solution de revêtement pour la fabrication d'un cathéter à ballonnet revêtu selon l'une des revendications 1 à 9.

16. Procédé de fabrication d'un cathéter à ballonnet revêtu selon l'une des revendications 1 à 9, comprenant les étapes suivantes :
a) fournir une solution aqueuse d'un copolymère de PEG et de PVA ;
b) ajouter progressivement la solution aqueuse de l'étape a) à au moins un solvant organique supplémentaire miscible sans limites avec de l'eau afin d'obtenir une solution aqueuse organique ;
c) mélanger la solution aqueuse organique avec un principe pharmaceutiquement actif et homogénéiser le mélange en formant une solution de revêtement contenant un principe actif ;
d) ajouter optionnellement du DMSO dans une quantité allant jusqu'à 10 % en volume à la solution de revêtement contenant le principe actif de l'étape c) par rapport au volume total d'eau et de solvant organique ;
e) application de la solution de revêtement contenant le principe actif de l'étape c) ou d) sur une surface d'un cathéter à ballonnet, et séchage de la solution de revêtement en formant le cathéter à ballonnet revêtu.
